# EUROPEAN PATENT APPLICATION

(11) **EP 4 112 024 A1**
(43) Date of publication of application: **04.01.2023**
(21) Application number: 21761850.3
(22) Date of filing: 15.02.2021
(51) Int. Cl.: A61F 13/15, A61F 13/56

(54) **PACKAGING SHEET AND PACKAGED ABSORBENT ARTICLE**

(30) Priority: 27.02.2020 JP 2020031875
(71) Applicant: DAIO PAPER CORPORATION, Shikokuchuo-shi Ehime 799-0492 (JP)
(72) Inventor: YOSHIBA, Megumi, Sakura-shi, Tochigi 329-1411 (JP)
(74) Representative: Epping - Hermann - Fischer
(86) International application number: PCT/JP2021/005568
(87) International publication number: WO 2021/172075

(57) **Abstract**

In a packaging sheet for packaging an absorbent article, a first region including one end of the packaging sheet in the first direction is caused to overlap on an outer surface of a second region including another end in the first direction to enable packaging of the absorbent article, and a sealing tape is adhered over a first portion of an outer surface of the first region and a second portion of the outer surface of the second region; and, a non-contact portion that does not contact the sealing tape is formed on the outer surface of the second portion.

## Description

### TECHNICAL FIELD

The present invention relates to a packaging sheet and a packaged absorbent article.

### BACKGROUND OF THE INVENTION

In general, absorbent articles such as sanitary napkins, panty liners, and incontinence pads are provided packaged in a packaging sheet for hygiene and for convenience of portability.

As such packaged absorbent articles (packaged absorbent articles or individual packages of absorbent articles), for example, Patent Document 1 describes individual packages in which overlapping portions are formed so that the leading edge is positioned outside of the packaging sheet and the packaging sheets are peelably bonded to each other at the side edges, the proximal end of the tape is secured to the surface of the packaging sheet located outside of the overlapping portions, and the free end of the tape is peelably bonded to the outer surface of the packaging sheet adjacent to the edge beyond one edge.

Patent Document 1 discloses that the surface of the individual package is smoothed so that the proximal side of the tape is secured to the non-woven packaging sheet.

### RELATED-ART DOCUMENT

### Patent Document

Patent Document 1: Japanese Patent Application Laid-Open No. 2003-175990

### SUMMARY OF THE INVENTION

### Problems to be Solved by the Invention

However, in the individual package described in Patent Document 1, the outer surface of the packaging sheet to which the free end side of the tape is attached is not specially modified. Therefore, when a highly adhesive tape is used, it may be difficult to peel off the free end side of the tape when the package is opened. If the tape, which is difficult to peel off on the free end side, is forcibly peeled off with strong force, the packaging sheet may break.

On the other hand, in order to improve the ease of peeling of the tape, if a tape with a low adhesive force is used, the tape may easily peel off even at the proximal end, so that the tape may fall off before opening the tape and become unable to serve as a tape.

One aspect of the invention is to provide a packaging sheet of which the tape is easy to peel off the tape when the tape is opened while maintaining its original function as a tape.

### Means for Solving the Problems

According to one aspect of the present invention, in a packaging sheet for packaging an absorbent article, a first region including one end of the packaging sheet in the first direction is caused to overlap on an outer surface of a second region including another end in the first direction to enable packaging of the absorbent article, and a sealing tape is adhered over a first portion of an outer surface of the first region and a second portion of the outer surface of the second region; and, a non-contact portion that does not contact the sealing tape is formed on the outer surface of the second portion.

### Effects of the Invention

According to an aspect of the invention, a packaged absorbent article may be provided with a tape that is easy to peel off upon opening, while maintaining its original function as a tape.

### BRIEF DESCRIPTION OF THE DRAWINGS

FIG. 1 is a planar view of a packaged absorbent article including a packaging sheet and an absorbent article according to an embodiment of the present invention;
FIG. 2 is a cross-sectional view of a line I-I in FIG. 1;
FIG. 3 is a planar view after partially unfolding the package absorbent article illustrated in FIG. 1;
FIG. 4 is a planar view of the unfolded state of the packaging sheet illustrated in FIG. 1 as seen from the side on which the absorbent article is placed;
FIG. 5 is an enlarged view of a second portion and its vicinity, of a second region of the packaging sheet illustrated in FIG. 1;
FIG. 6 is a partially enlarged view of the cross-section of a line II-II in FIG. 1;
FIG. 7 is a diagram illustrating a modified example of non-contact portions (recessed portions);
FIG. 8 is a diagram illustrating another modified example of non-contact portions (recessed portions);
FIG. 9 is a diagram illustrating yet another modified example of non-contact portions (recessed portions);
FIG. 10 is a diagram illustrating yet another modified example of non-contact portions (recessed portions);
FIG. 11 is a planar view of a packaged absorbent article including a packaging sheet and an absorbent article in a modified example;
FIG. 12 is an enlarged view of a second portion and its vicinity, of the second region of the packaging sheet illustrated in FIG. 11; and
FIG. 13 is a partially enlarged view of the cross-section of a line III-III in FIG. 11.

### DETAILED DESCRIPTION OF THE INVENTION

Hereinafter, embodiments of the present invention will be described with reference to the drawings. In each drawing, unless otherwise noted, the description of the same or corresponding structure may be omitted with the same reference numerals. Also, the drawings are schematic to aid in understanding the invention.

### (Packaged Absorbent Articles)

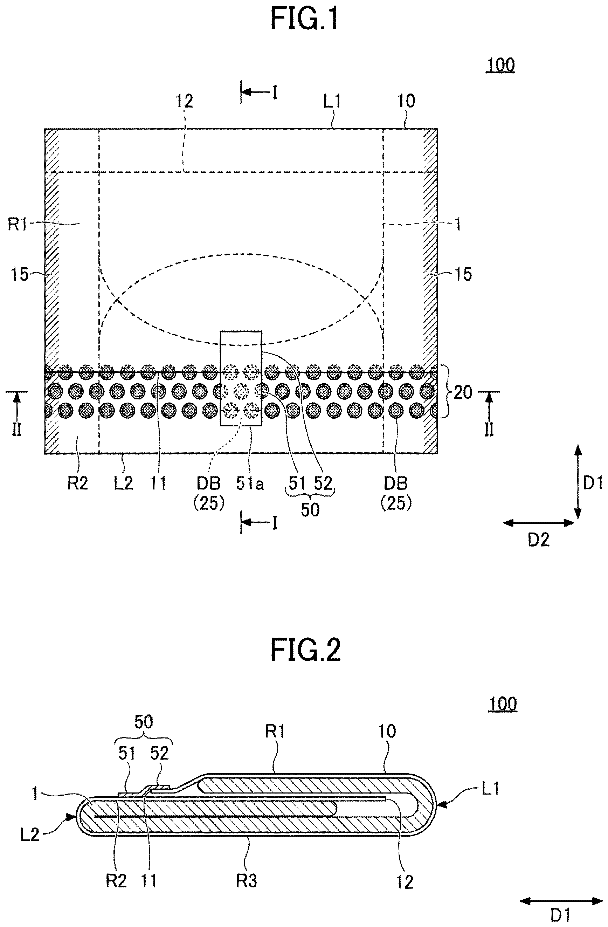

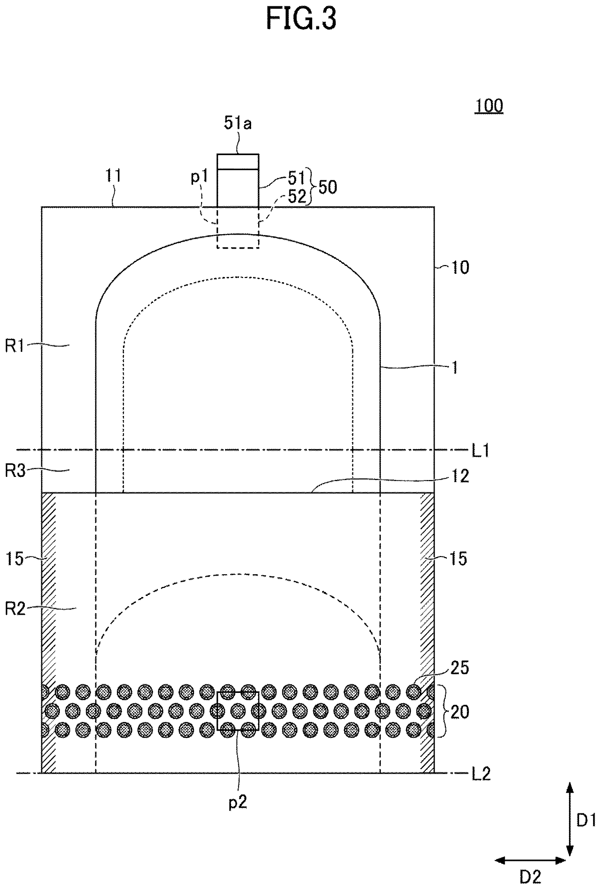

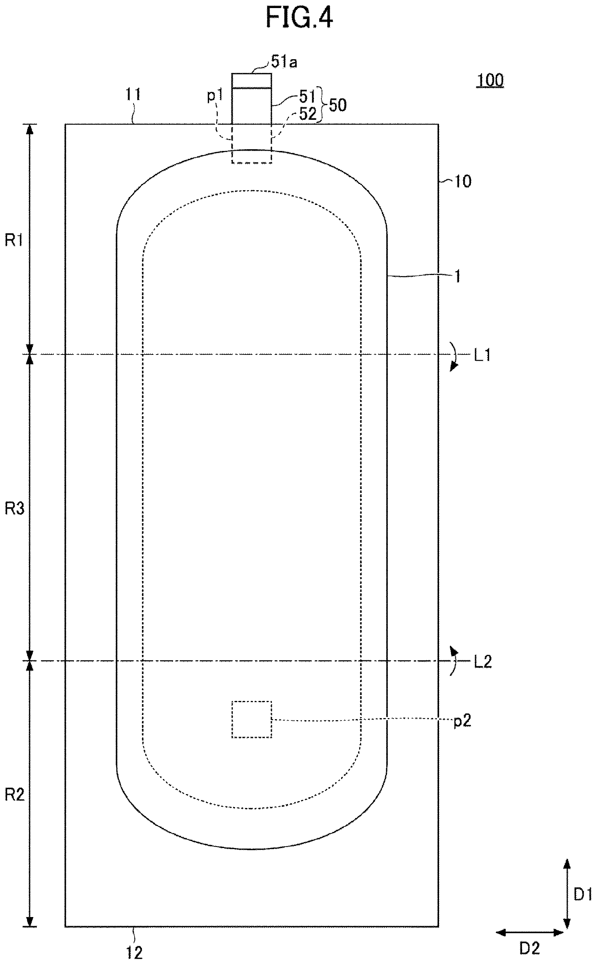

FIG. 1 is a planar view of a packaged absorbent article 100 including a packaging sheet 10 and an absorbent article 1 according to an embodiment of the present invention. FIG. 2 is a cross-sectional view of the I-I line in FIG. 1. FIG. 3 is a planar view after partially unfolding the package absorbent article 100 illustrated in FIG. 1. FIG. 4 is a planar view of the unfolded state of the packaged absorbent article 100 illustrated in FIG. 1 as seen from outside of the packaging sheet 10.

As illustrated in FIGS. 1 to 4, the packaged absorbent article 100 includes the packaging sheet 10 and the absorbent article 1 packaged by the packaging sheet 10. In other words, the packaged absorbent article 100 is an individual package containing one absorbent article 1 in one packaging sheet 10. The packaged absorbent article 100 may be obtained by placing the absorbent article 1 on the unfolded packaging sheet 10, and folding the packaging sheet 10 with the absorbent article 1 so that the areas on both sides in one direction of the packaging sheet 10 overlap each other, that is, folding the packaging sheet 10 to the side on which the absorbent article 1 is placed, and thereby, folding the packaging sheet 10 with the absorbent article 1 in at least three.

More specifically, the packaging sheet 10 includes a first region R1 including one end 11 in the longitudinal direction (first direction) D1 of the packaging sheet 10 and a second region R2 including another end 12 in the longitudinal direction D1 of the packaging sheet 10, as illustrated in FIGS. 3 and 4. Then, the absorbent article 1 can be wrapped by overlapping the first region R1 on the outer surface of the second region R2. At this time, the first region R1 is folded at a first folding line L1 extending along a shorter direction (second direction) D2 perpendicular to the longitudinal direction D1. The second region R2 is folded at a second folding line L2 extending along the shorter direction D2 before the first region R1 is folded back. In the present embodiment, the region between the first region R1 and the second region R2 is defined as a third region R3.

When the second region R2 is folded at the second folding line L2, the other end 12 in the longitudinal direction D1 may be folded so as to reach the first folding line L1 of the first region R1, or the other end 12 may be folded so as to be at a position away from the first folding line L1, as illustrated in FIG. 1. When folding the first region R1 at the first folding line L1, it is preferable that the one end 11 of the packaging sheet 10 in the longitudinal direction D1 does not extend beyond the second folding line L2 and does not extend beyond the second region R2. Such a packaging method of overlapping two areas at the ends of the packaging sheet 10 is simple and is preferred because the absorbent article can be securely enclosed without exposure to the external environment.

### (Absorbent Articles)

The absorbent article 1 used in embodiments of the present invention may be a sanitary napkin, panty liner, light incontinence pad, and the like. As used herein, the absorbent article 1 has an elongated shape in a planar view, but the planar shape of the absorbent article 1 is not limited to that illustrated. The absorbent article 1 may be, for example, a fluid-absorbing pad in a shape that is not folded together with the packaging sheet but is wrapped by the packaging sheet, such as a regular polygon including a square, a circle, or the like. If the absorbent article 1 is an elongated shape, the total length of the absorbent article 1 can be 140 to 420 mm and the width of the absorbent article 1 can be 50 to 110 mm.

The absorbent article 1 may have a structure in which a liquid impermeable back sheet, an absorbent article, and a liquid permeable surface sheet are laminated in this order. The back sheet may be a sheet material having at least water shielding properties, such as a sheet of olefin resin, such as polyethylene or polypropylene. A laminated non-woven fabric made by laminating a non-woven material with a polyethylene sheet or the like or a non-woven laminated sheet made by substantially securing impermeability by interposing a waterproof film with the laminated material or the like can be used. A material permeable to moisture may also be used.

As the surface sheet, a porous or non-porous non-woven fabric, a porous plastic sheet, or the like is preferably used. As the material fibers constituting the non-woven fabric, for example, synthetic fibers such as polyethylene, olefin such as polypropylene, polyester, polyamide, and the like; regenerated fibers such as rayon, cupra, and the like; and blended fibers, as well as natural fibers such as cotton and the like, can be used singly or in a combination of two or more materials.

The absorbent material is preferably, but is not limited to, a material capable of absorbing and retaining bodily fluids, including cotton pulp and water absorbent polymers. As the water absorbent polymer, superabsorbent polymer (SAP), superabsorbent fiber (SAF), and a combination thereof can be used. The pulp may be a chemical pulp obtained from wood, a cellulosic fiber such as dissolved pulp, or an artificial cellulose fiber such as rayon, acetate, and the like.

The thickness of the absorbent article may be in the range of 0.5 to 25 mm, preferably in the range of 1.5 to 6.5 mm. The absorbent article can also have an inflated structure in which the area corresponding to the fluid discharge (fluid discharge corresponding area) and the area facing the groove of the buttocks behind the area corresponding to the fluid discharge are located.

The absorbent article has a dimension and shape that does not protrude from the surface sheet and the back sheet. At the front and rear edges of the absorbent article, the outer edges of the back sheet and the surface sheet are bonded by adhesives such as hot melt and the like; adhesive methods such as heat seal, ultrasonic seal, and the like. Further, a side non-woven fabric may be provided along the longitudinal direction at both ends of the side of the absorbent article in the width direction. As the side non-woven fabric, a water-repellent treated non-woven fabric or a hydrophilic treated non-woven fabric may be used.

### (Packaging Sheet)

Although the shape of the packaging sheet 10 is not particularly limited, the packaging sheet 10 preferably has an elongated shape, for example, a rectangle, an elongated ellipse, or the like, in an unfolded state as illustrated in FIG. 4. This allows for hygienic packaging of the elongated and planar absorbent article 1.

The dimensions of the packaging sheet 10 depend on the size and shape of the absorbent article 1 to be packaged, for example, the length in the longitudinal direction D1 (sometimes simply referred to as the length) may be 100 to 450 mm with the packaging sheet 10 being unfolded, and the length in the shorter direction D2 (sometimes simply referred to as the width) perpendicular to the longitudinal direction D1 may be 70 to 250 mm.

When a relatively long absorbent article is packaged, a longer length packaging sheet 10 may be used to match the length of the absorbent article. In this case, the length of the second region R2 may be longer than the length of the third region R3, and when the second region R2 is folded, first, the second region R2 is folded to the side on which the absorbent material is placed at the third folding line extending along the shorter direction D2, which is located between the second folding line L2 and the other end 12. Subsequently, the entire second region R2 is folded at the second folding line L2, thereafter, the first region R1 is folded at the first folding line L1 to obtain the packaged absorbent article in which the absorbent article with the packing sheet is folded in four in total.

The material constituting the packaging sheet 10 is not particularly limited and may be a sheet containing fibers such as paper, non-woven fabric, or the like, and may be a resin film.

When paper is used for the packaging sheet 10, paper is preferable in that paper imparts a unique texture with less environmental burden at the time of disposal. As used herein, the term "paper" can refer to plant fibers or other fibers that are glued together with an adhesive to form a flat plate. In particular, a material made mainly from plant fibers (pulp), for example, a material containing 50% or more of plant fibers, preferably 80% or more, may be used. The pulp types included in the paper may include wood pulp, non-wood pulp, and recycled paper pulp, which may be either mechanical pulp or chemical pulp. Additives may be added to the paper. Additionally, specific paper examples used in the packaging sheet 10 include various types of paper, such as standard paper, Japanese paper, processed paper, synthetic paper, and the like. Also, paper which is conventionally used for other purposes, such as newspaper, printing paper (including high quality paper), writing paper, drawing paper, packaging paper, laminated paper, miscellaneous paper, and the like, can be used either raw or processed.

When paper is used as the material of the packaging sheet 10, the packaging sheet 10 may be a lowdensity tissue paper to facilitate folding and maintaining the condition after packaging the absorbent articles. The thin paper may be tissue paper, Indian paper, rice paper (used as cigarette rolls or the like), glassine paper, tissue paper, toilet paper, filter paper, and the like. The packaging sheet 10 may also be processed after papermaking. Examples of the above-described processing include creping, embossing, calendaring, water-repellent processing (including formation of a water-repellent coating layer), slit processing, ply processing, printing processing, and the like. Creping and embossing can improve both strength and flexibility. In addition, in the case of the water-repellent processing of the packaging sheet 10, for example, a water-repellent agent containing a silicone-based resin, a paraffin-based resin, a fluorine-based resin, or the like may be applied to at least one of the outer surface (the side exposed to the outside when the absorbent article 1 is packaged) of the packaging sheet 10 and the inner surface opposite to the outer surface thereof.

When paper is used as the material of the packaging sheet 10, the density of the packaging sheet may be preferably 50 g/m² or less, more preferably 40 g/m² or less, further preferably 30 g/m² or less, 20 g/m² or less, 11 g/m² or less, or 8 g/m² or less. In addition, the lower limit of the density of the packaging sheet 10 is not particularly limited as long as it functions as the packaging sheet 10, but may be preferably 5 g/m² or more, and more preferably 10 g/m² or more. The thickness of the packaging sheet 10 made by paper may also be preferably 40 to 200 µm, more preferably 100 to 200 µm.

When a non-woven material is used for the packaging sheet 10, the non-woven material can improve the texture, the soft feel, or the like of the packaging sheet. Therefore, using the non-woven material is preferable. The non-woven fabric used is preferably a melt-blown non-woven fabric or a spunbonded non-woven fabric, and a plurality of layers of these non-woven fabrics may be laminated together. The non-woven fabric may be a polyolefin such as polypropylene, polyethylene, and the like; polyamide such as polyester, nylon, and the like.

When a resin film is used as the packaging sheet 10, the resin to be used may include polyolefins such as polypropylene, polyethylene; polyester, polyvinyl alcohol, and the like. As the resin film, the resin is preferably stretched. The resin film may also be a non-breathable film or a breathable film. The use of a resin film improves the design of the packaging sheet by providing a clear outline of the print and a good color development of the colorant.

As the packaging sheet 10, one packaging sheet 10 may be a single layer sheet made of the material described above, or one packaging sheet 10 may be a laminated sheet made of a plurality of layers made of different materials. Thus, for example, the packaging sheet may be configured by laminating a resin film to the paper sheet described above.

The packaged absorbent article 100 is provided with peelable seal portions 15 and 15 at both edges in the shorter direction D2, as illustrated in FIG. 1. The seal portions 15 and 15 prevent the absorbent article 1 from being exposed to the outside and also prevent dust and the like from entering the inside of the packaged absorbent article 100. The seal portions 15 and 15 may be formed, for example, by heat sealing, ultrasonic sealing, or by adhesives.

### (Sealing Tape)

In the packaged absorbent article 100, the absorbent article 1 is packaged with the packaging sheet 10, followed by attaching the sealing tape 50 (FIGS. 1 to 4). The sealing tape 50 is an adhesive tape having an adhesive layer on the side where the packaging sheet 10 is provided. The adhesive tape is a tape that can be detached from the outer surface of the packaging sheet 10 even after some time has passed.

The sealing tape 50 is applied from the first region R1 that is overlapped on the outer surface of the second region R2 to the second region. In other words, the sealing tape 50 is attached to the outer surface of the packaging sheet 10 so as to span the one end 11 in the longitudinal direction D1 of the packaging sheet 10 included in the first region R1. By providing the sealing tape 50, the first region R1 may be at least partially secured to the second region R2 so that the first region R1 does not open. Therefore, it is possible to prevent dust and the like from entering between the first region R1 and the second region R2 before opening the packaged absorbent article 100. Further, it is possible to prevent the first region R1 from peeling off the second region R2 due to a hand or an object accidentally entering between the first region R1 and the second region R2.

When the packaged absorbent article 100 is opened, normally, the vicinity of the second folding line L2 of the packaging sheet 10 is held by one hand and a portion (that is a free portion 51) attached to the second region R2 of the sealing tape 50 is held by the other hand to pull the packaged absorbent article 100 off the sealing tape 50 from the second region R2. Then, when the sealing tape 50 is pulled further, the sealing of the sealing portions 15 and 15 is released while a portion attached to the first region R1 of the sealing tape 50 (that is a securing portion 52) is kept attached to the first region R1. Thus, by continuing to pull the sealing tape 50, the first region R1 can be lifted with the sealing tape 50 and peeled off from the second region R2 to develop the packaged absorbent article 100.

The portion to which the sealing tape 50 is attached in the first region R1 is designated as a first portion p1, and the portion to which the sealing tape 50 is attached in the second region R2 is designated as a second portion p2. In other words, in the packaging sheet 10, the portion to which the free portion 51 of the sealing tape 50 is attached is the first portion p1, and the portion to which the securing portion 52 of the sealing tape 50 is attached is the second portion p2. The length of the second portion p2 in the longitudinal direction D1 may be 60 to 150% of the length of the first portion p1 in the longitudinal direction D1.

An end 51a at the side of the free portion 51 of the sealing tape 50 may not be attached to the packaging sheet 10. For example, as illustrated in FIGS. 1, 3, and 4, the end of the free portion 51 of the sealing tape 50 may be folded back and the end 51a on which the outer surface of the sealing tape 50 (the surface without the adhesive layer) faces the packaging sheet 10 may be formed. This allows a user to easily lift the free portion 51 of the sealing tape 50 by pinching the end 51a when the packaged absorbent article 100 is opened.

The width of the sealing tape 50 may be preferably 5 to 30 mm, more preferably 10 to 20 mm. The overall length of the sealing tape 50 may also be preferably 10 to 50 mm, and more preferably 15 to 40 mm.

### (Non-Contact Portions)

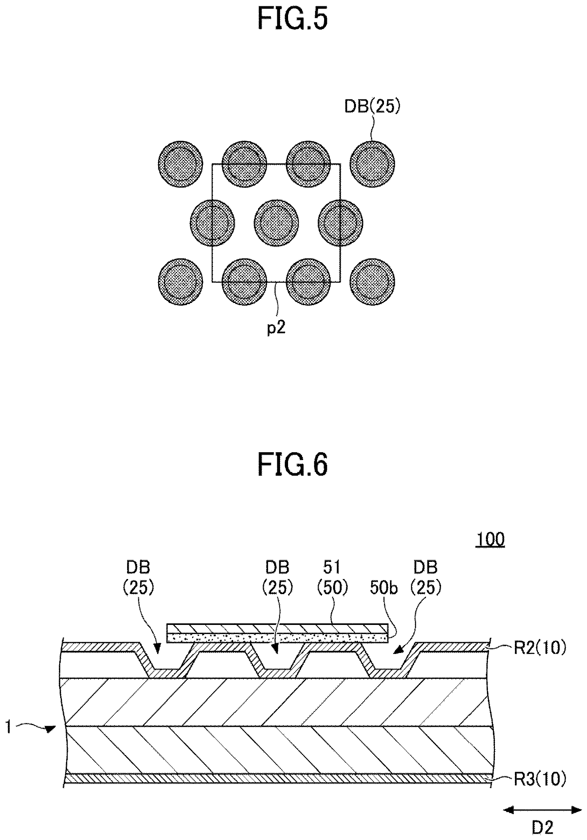

As illustrated in FIGS. 1 and 3, in the present embodiment, non-contact portions 25 are formed on the outer surface of the second portion p2, where the sealing tape 50 is attached in the second region R2. Further, FIG. 5 illustrates an enlarged view of the second portion p2 and its vicinity, in the packaging sheet 10 illustrated in FIG. 3. Also, FIG. 6 illustrates a partially enlarged view of the cross-section of the line II-II in FIG. 1.

In the packaged absorbent article 100, in the state in which the sealing tape 50 is adhered to the packaging sheet 10, the adhesive layer provided on the sealing tape 50 contacts with the outer surface of the packaging sheet 10. However, when the non-contact portions 25 are provided on the outer surface of the second portion p2 of the second region R2, an adhesive layer 50b of the free portion 51 of the sealing tape 50 does not contact or does not easily contact with the packaging sheet 10 (FIG. 6). Thus, in the second portion p2, the contact area between the sealing tape 50 and the packaging sheet 10 is reduced and the bonding force between the sealing tape 50 and the packaging sheet 10 is reduced. Thus, when the packaged absorbent article 100 is opened, the force required to peel off the free portion 51 of the sealing tape 50 from the outer surface of the second region R2 can be reduced, and the packaged absorbent article 100 can be opened with smooth operation.

Further, in the prior art in which the non-contact portions 25 are not formed in the second portion p2, a sealing tape 50 having low adhesion has been used in order to facilitate peeling of the free portion 51 of the sealing tape 50. However, if the adhesion of the entire sealing tape 50 is weakened, the adhesion of the securing portion 52 of the sealing tape 50 is also weakened, and the securing portion 52 of the sealing tape 50 may peel off from the first region R1 prior to opening or during the opening operation of the packaged absorbent article 100, so that the sealing tape 50 may not fulfill its original role of preventing the opening of the first region R1. On the other hand, according to the present embodiment, the bonding force can be reduced only in the free portion 51 of the sealing tape 50 without changing the configuration of the sealing tape 50, and the free portion 51 can be easily peeled while maintaining the bonding force of the securing portion 52 of the sealing tape 50 to the first region R1.

In addition, when users often change absorbent articles, the users often wrap used absorbent articles and dispose of them using the packaging sheets 10 that are removed when new packaged absorbent articles are opened. At this time, after the used absorbent article is rolled up and wrapped in the packaging sheet 10, the sealing tape 50 that remains attached to the first region R1 of the packaging sheet 10 can be reused to retain the packaged absorbent article. More specifically, the used absorbent article is wrapped so that the first region R1 having the sealing tape 50 of the packaging sheet 10 is positioned outward, and the free portion 51 of the sealing tape 50 is affixed to the surface of the packaging sheet 10 disposed beneath the free portion 51. This ensures that the used absorbent articles remain compactly rolled up and wrapped. Accordingly, it is preferable that the free portion 51 of the sealing tape 50, once peeled off at the time of opening, can be reusable (having re-sealability).

Particularly, when the packaging sheet 10 is a fiber-containing sheet, the fibers on the surface of the packaging sheet 10 easily adhere to the adhesive layer of the sealing tape 50 when the sealing tape 50 is peeled off when the packaged absorbent article is opened. The fibers adhered to the peeled sealing tape 50 may then reduce the adhesiveness of the adhesive layer. On the other hand, according to the present embodiment, the non-contact portions 25 are formed in the second portion p2 to which the free portion 51 of the sealing tape 50 is attached, the fibers do not adhere to the non-contact portions 25 or do not easily adhere thereto. Therefore, it is possible to improve the adhesion of the free portion 51 of the sealing tape 50 after opening (after peeling off the sealing tape 50). Accordingly, when the used absorbent article is disposed of and the used absorbent article is wrapped in the packaging sheet 10 and secured with the sealing tape 50, the sealing tape 50 can be more securely fastened to maintain the used absorbent article enclosed therein.

As illustrated in FIGS. 1, 3, 5, and 6, the non-contact portions 25 can be formed, for example, by forming recessed portions DB. The recessed portions DB may be recessed portions of the packaging sheet 10 formed by debossing from the outside to the inside of the packaging sheet 10. The recessed portions DB are portions that are lower in height than the surrounding area, and formed, for example, by pressing using a press machine having a combination of male and female dies, with the male die positioned on the outer surface of the packaging sheet 10. The formation of such recessed portions DB can be performed while heating using a heating device or the like, or without using a heating device or the like.

In the example illustrated in FIG. 6, the recessed portion DB is configured so that the inner surface of the recessed portion DB does not come into contact with the sealing tape 50. However, depending on the material of the packaging sheet 10, the dimensions of the recessed portion DB (planar view dimensions and depth), or the like, the inner surface of the recessed portions DB may come into contact with a part of the sealing tape 50.

As illustrated in FIGS. 1, 3, 5, and 6, a plurality of recessed portions DB may be provided. The plurality of recessed portions DB may be formed spaced apart from each other, particularly as illustrated in FIG. 5. The plurality of spaced apart recessed portions DB allows for the distributed presence of areas where the sealing tape 50 and the packaging sheet 10 do not or are unlikely to come into contact with each other. Accordingly, the sealing tape 50 can be smoothly peeled off even if the force is biased when a user peels the free portion 51.

The non-contact portions 25 are formed on the outer surface of at least the second portion p2. That is, the non-contact portions 25 may be provided in a region other than the second portion p2 of the second region R2. For example, as illustrated in FIGS. 1 and 3, a plurality of discontinuous recessed portions DB forming the non-contact portions 25 may be provided in the area (non-contact portion forming region 20) extending over a predetermined length in the longitudinal direction D1 and over the length (width) in the shorter direction D2. As such, the packaging sheet 10 in which the non-contact portions 25 are formed in a strip-like area throughout the shorter direction D2 is preferably manufactured using a press machine having a pair of rolls for conveying the packaging sheet 10 along the shorter direction D2. Further, since a plurality of recessed portions DB are formed in the strip-like non-contact portion forming region 20, the design characteristics can be improved.

Also, as illustrated in FIGS. 1, 3, and 5, a plurality of recessed portions DB are spaced apart from each other in both the longitudinal direction D1 and the shorter direction D2. That is, the plurality of recessed portions DB are arranged in a distributed manner in both the longitudinal direction D1 and the shorter direction D2. Thus, such configuration can be more responsive to the bias of the force applied by the user in peeling off the free portion 51.

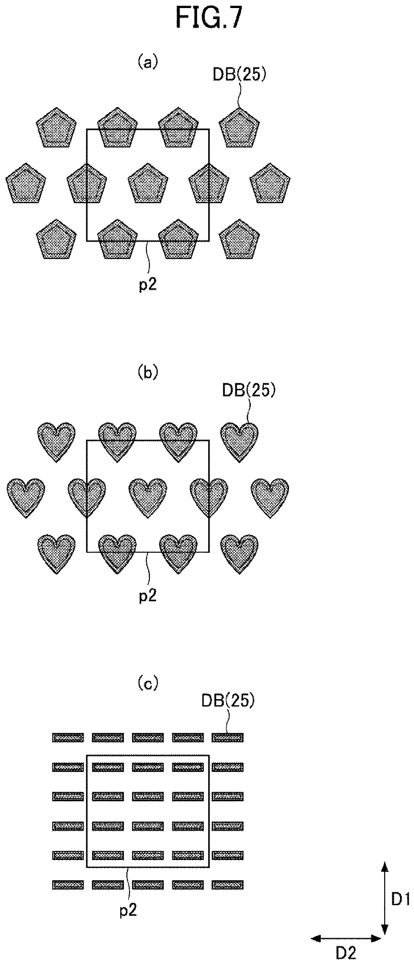

As in the present example, when the plurality of recessed portions DB are spaced apart from each other in the longitudinal direction D1 and the shorter direction D2, and the plurality of non-contact portions 25 are spaced apart from each other in the longitudinal direction D1 and the shorter direction D2, the area of planar view of a single recessed portion DB, that is, the area of planar view of the portion that is depressed from the periphery, is preferably 0.75 to 180 mm², more preferably 7 to 20 mm². The distance between the recessed portions DB may be 1 to 5 mm. The arrangement of the recessed portions DB spaced apart from each other in the longitudinal direction D1 and the shorter direction D2 may be grid-like (FIG. 7(c)) or a staggered pattern (FIG. 1, 3, 5, and the like).

As illustrated in FIGS. 1, 3, and 5, the planar view shape (the shape of the contour of the portion deeper than the surrounding area) of each recessed portion DB may be a circular. Thus, when the planar view shape of the recessed portion DB is circular, the diameter of the circle may preferably be 1 to 15 mm, and more preferably 3 to 5 mm.

The ratio of the total of the planar view area of the non-contact portions 25 to the area of the second portion p2 may preferably be 10 to 70%, and more preferably 20 to 50%, depending on the adhesive performance of the sealing tape 50 and the material of the packaging sheet 10. In particular, when the ratio of the total of the planar view area of the non-contact portions 25 is set to 50% or less, the contact area can be set to 50% or more. Therefore, the role of the sealing tape 50 to prevent the first region R1 from being opened before opening the seal can be secured.

In addition, if the bonding force between the securing portion 52 of the sealing tape 50 and the first portion p1 is not hindered, and the securing portion 52 is not detached from the first region R1 before or when the sealing is opened, the non-contact portions may also be formed in the first portion p1 of the first region R1. However, since the bonding force between the securing portion 52 and the first portion p1 is preferably higher, the non-contact portions are not preferably formed on the first portion p1. Further, the non-contact portions 25 are not preferably formed on the outer surface of the packaging sheet 10 in the region other than the non-contact portion forming region 20. Accordingly, when the used absorbent article is rolled up, wrapped, and disposed in the packaging sheet 10, the sealing tape 50 can be adhered to the outer surface of the packaging sheet well after packaging, thereby maintaining a compact state in which the used absorbent article is rolled up.

### (Modified Examples of the Shapes of Recessed portions)

When the recessed portions DB are disposed discontinuously in both the longitudinal direction D1 and the shorter direction D2, the planar view shape of the recessed portion DB is not limited to circular, but may be any shape, such as an ellipse; a polygon such as a triangle, a square, or the like; a heart shape, a star shape, a drop shape, and the like. FIG. 7 illustrates modified examples of the planar view shapes of the recessed portions DB. FIG. 7(a) illustrates an example in which the planar view shape of the recessed portion DB is an equilateral pentagon shape, and FIG. 7(b) illustrates an example in which the planar view shape of the recessed portion DB is a heart shape. FIG. 7(c) illustrates an example in which the planar view shape of the recessed portion DB is rectangular. FIGS. 7(a) to (c) are diagrams corresponding to FIG. 5 and are enlarged views of the second portion p2 and its vicinity, of the second region R2 in a state in which the first region R1 is not overlapped.

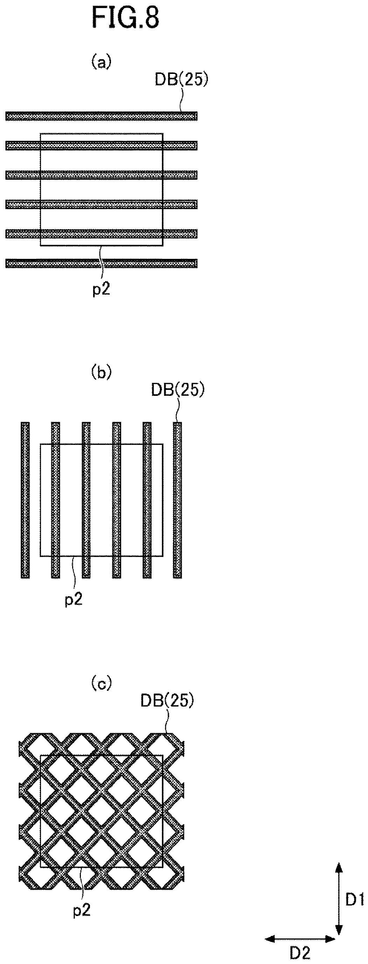

Another modified example of the recessed portions DB are illustrated in FIG. 8. FIGS. 8(a) to (c) are diagrams corresponding to FIGS. 5 and 7, and are enlarged views of the second portion p2 and its vicinity, of the second region R2 in a state in which the first region R1 is not overlapped.

In the examples illustrated in FIG. 8, the recessed portions DB are formed in a linear or grooved pattern. For example, in the example illustrated in FIG. 8(a), a plurality of linear recessed portions DB extending in the shorter direction D2 are spaced apart in the longitudinal direction D1. In the example illustrated in FIG. 8(b), a plurality of linear recessed portions DB extending in the longitudinal direction D2 are spaced apart in the shorter direction D2. Further, in the example illustrated in FIG. 8(c), a meshwork of non-contact portions 25 is formed in which a plurality of linear recessed portions DB extending along one direction and a plurality of linear recessed portions DB extending along a different direction intersecting the one direction are intersected. In the example of FIG. 8(c), a meshwork of non-contact portions 25 is formed in which a plurality of linear recessed portions DB along one direction extending obliquely with respect to the longitudinal direction D1 or the shorter direction D2 and a plurality of linear recessed portions DB along the other direction substantially perpendicular to the one direction are intersected.

When the recessed portions DB are formed in a linear or groove pattern as in the example illustrated in FIG. 8, the width of the line may preferably be 2 to 5 mm, and more preferably 2 to 3 mm. Furthermore, the linear recessed portions DB preferably extend from one edge of the second portion p2 to another edge.

### (Modified Examples in the Arrangement of Recessed portions)

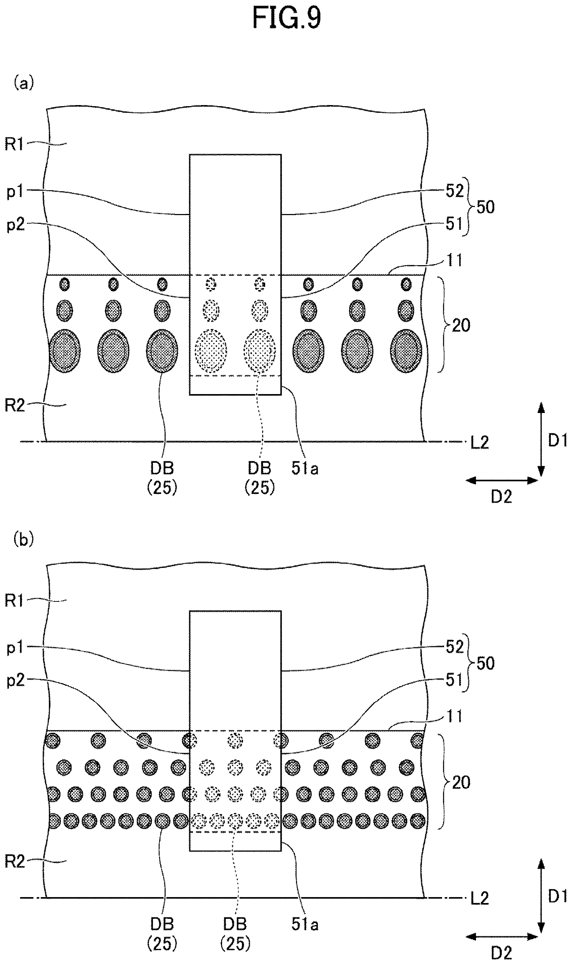

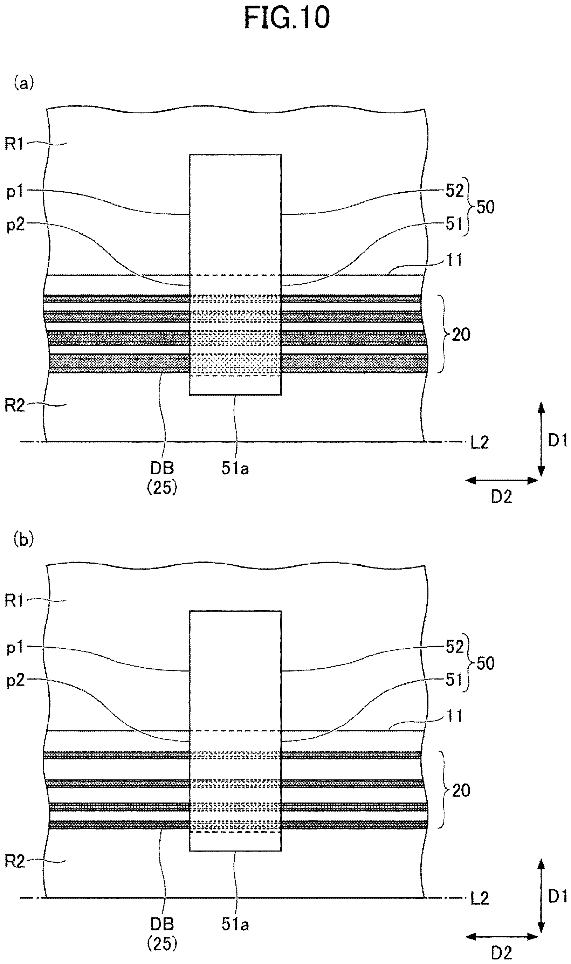

In addition, FIGS. 9 and 10 illustrate modified examples of the arrangement of the recessed portions DB in the non-contact portion forming region 20. FIG. 9 illustrates an example in which at least a plurality of recessed portions DB are formed discontinuously along both the longitudinal direction D1 and the shorter direction D2 in at least a second portion p2 in the similar manner as in FIGS. 1 to 8. However, the examples illustrated in FIGS. 1 to 8 are different in that the configuration of the recessed portions DB is not uniform across the non-contact portion forming region 20.

For example, in the example illustrated in FIG. 9(a), circular or elliptical recessed portions DB are disposed discontinuously along the longitudinal direction D1 and the shorter direction D2, but the planar view area of one recessed portion DB varies depending on the positions in the longitudinal direction D1. More specifically, in the packaged absorbent article 100 (with the packaging sheet 10 folded), the planar view area of the recessed portion DB on the side (the lower side in FIG. 9) away from the one end 11 included in the first region R1 of the packaging sheet is larger than the planar view area of the recessed portion DB on the side (the upper side in FIG. 9) close to the one end 11. In other words, the planar view area of the recessed portion DB on the side away from the other end 12 included in the second region R2 of the packaging sheet 10 is larger than the planar view area of the recessed portion DB on the side close to the other end 12. In addition, the planar view area of the recessed portion DB near the second folding line L2 is larger than the planar view area of the recessed portion DB on the distal side of the second folding line L1.

Further, in the example illustrated in FIG. 9(b), a plurality of circular recessed portions DB is disposed discontinuously along the longitudinal direction D1 and the shorter direction D2, and each recessed portion DB has the same planar view shape and planar view area, but the distance of the shorter direction D2 between the recessed portions DB varies depending on the positions in the longitudinal direction D1. More specifically, in the packaged absorbent article 100, the distance between the plurality of recessed portions DB in the shorter direction D2 on the side (the lower side in FIG. 9) away from the one end 11 included in the first region R1 of the packaging sheet 10 is shorter than the distance between the plurality of recessed portions DB in the shorter direction D2 on the side close to the one end 11 (the upper side in FIG. 9). In other words, the distance between the plurality of recessed portions DB in the shorter direction D2 on the side away from the other end 12 included in the second region R2 of the packaging sheet 10 is shorter than the distance between the plurality of recessed portions DB in the shorter direction D2 on the side close to the other end 12. In addition, the distance between the plurality of recessed portions DB in the shorter direction D2 near the second fold line L2 is smaller than the distance between the plurality of recessed portions DB in the shorter direction D2 on the distal side from the second fold line L2. In other words, the plurality of recessed portions DB on the side away from the one end 11 included in the first region R1 of the packaging sheet 10 are denser than the plurality of recessed portions DB on the side close to the one end 11.

In the example illustrated in FIG. 10(a), a plurality of linear recessed portions DB extending in the shorter direction D2 is disposed spaced apart in the longitudinal direction D1, but the width of the plurality of linear recessed portions DB differs depending on the positions in the longitudinal direction D1. More specifically, the width of the recessed portion DB at a side (the lower side in FIG. 10) away from the one end 11 included in the first region R1 of the packaging sheet 10 is greater than the width of the recessed portion DB at a side (the upper side in FIG. 10) close to the one end 11. In other words, the width of the recessed portion DB on the side away from the other end 12 included in the second region R2 of the packaging sheet 10 is greater than the width of the recessed portion DB on the side close to the other end 12.

In the example illustrated in FIG. 10(b), a plurality of linear recessed portions DB extending in the shorter direction D2 is disposed spaced apart in the longitudinal direction D1, but the distance along the longitudinal direction D1 between the linear recessed portions DB varies depending on the positions in the longitudinal direction D1. More specifically, the distance in the longitudinal direction D1 between the plurality of recessed portions DB at a side (the lower side in FIG. 10) away from the one end 11 included in the first region R1 of the packaging sheet 10 is shorter than the distance in the longitudinal direction D1 between the plurality of recessed portions DB at a side (the upper side in FIG. 10) close to the one end 11. In other words, the distance between the plurality of recessed portions DB on the side away from the other end 12 included in the second region R2 of the packaging sheet 10 is shorter than the distance between the plurality of recessed portions DB on the side close to the other end 12.

FIGS. 9(a) and 9(b) and FIGS. 10(a) and 10(b) are different modified examples, but in both cases, the area of the non-contact portions 25 per unit area on the side (the lower side in FIGS. 9 and 10) away from the one end 11 included in the first region R1 of the packaging sheet 10 is larger than the area of the non-contact portions 25 per unit area on the side close to the one end 11 (the upper side in FIGS. 9 and 10) in the state that the absorbent article 1 is wrapped with the packaging sheet 10. The area of the non-contacting portion 25 per unit area can be gradually or progressively increased as the non-contacting portion 25 is disposed away from the one end 11 along the longitudinal direction D1. By this configuration, the contact area between the sealing tape 50 and the packaging sheet 10 can be smaller on the side away from the one end 11 included in the first region R1 of the packaging sheet 10, that is, on the side near the end 51a of the free portion 51 of the sealing tape 50. Therefore, at the beginning of peeling of the sealing tape 50, the force required to peel off can be reduced and the sealing tape 50 can be smoothly peeled off.

### (Modified Examples of Non-Contact Portions)

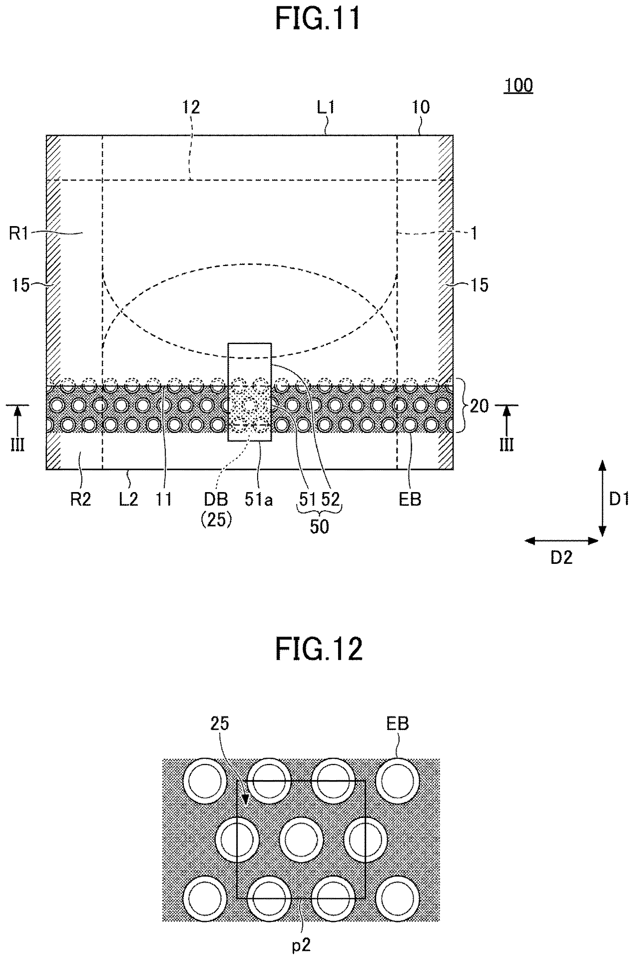

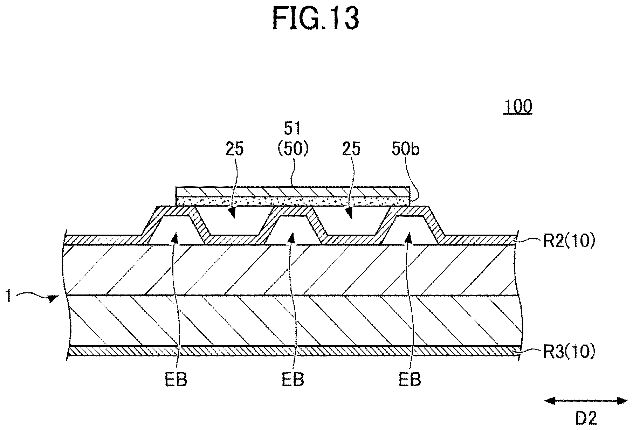

FIGS. 11 to 13 illustrate modified examples of the configuration of the non-contact portions 25. FIG. 11 illustrates a planar view of the packaged absorbent article 100. FIG. 12 illustrates an enlarged view of the second portion p2 and its vicinity, of the second region R2, and FIG. 13 illustrates a partially enlarged view of the cross-section of the line III-III in FIG. 11. The packaging sheet 10 illustrated in FIGS. 11 to 13 has the same basic configuration as the packaging sheet 10 illustrated in FIGS. 1 to 6, but differs in configuration from the non-contact portions 25. In the packaging sheet 10 illustrated in FIGS. 11 to 13, a plurality of discontinuous projecting portions EB is formed. A portion between the projecting portions EB becomes relatively deep with respect to the projecting portion EB to form the non-contact portions 25. In the example of FIGS. 11 to 13, the non-contact portions 25 are continuous in the second region p2.

The projecting portion EB may be a raised portion formed by embossing from the inner surface to the outer surface of the packaging sheet 10. The projecting portions EB may be formed, for example, by pressing using a press machine having a combination of a male and female dies, in a configuration opposite to that used to form the recessed portions DB, that is, the projecting portions EB can be formed by pressing so that male die is positioned on the inner surface side of the packaging sheet 10.

The projecting portions EB are formed at least in the second portion p2 in the same manner as in the recessed portions DB. That is, the projecting portions EB may be formed in the second portion p2 and the area other than the second portion, and the non-contact portions 25 may be formed in a portion other than the second portion p2. For example, the projecting portions EB are provided in a region (non-contact portion forming region 20) extending over a predetermined length in the longitudinal direction D1 and over the entire length (width) in the shorter direction D2, whereby the non-contact portions 25 are formed in the non-contact portion forming region 20 extending over a predetermined length in the longitudinal direction D1 and over the length (width) in the shorter direction D2.

The shape and dimensions of the projecting portions EB and the arrangement (such as the distance between the projecting portions EB) are not particularly limited if the non-contact portions 25 can be formed in the second region R2, and can be appropriately selected depending on the material of the packaging sheet 10, the adhesion of the adhesive layer of the sealing tape 50, and the like. For example, the plan view shape and dimensions of the projecting portions EB, as well as the arrangement or the like, can be formed in the same manner as in the planar view shape and dimensions of the recessed portions DB, as well as the distance between the recessed portions DB. Thus, the planar view shape of the projecting portion EB can be circular, elliptical, polygonal, heart shape, or the like. The planar view area of the projecting portion EB may also be preferably 0.75 to 180 mm² and more preferably 7 to 20 mm².

Even in the present examples in which the non-contact portion 25 is formed by forming a plurality of discontinuous embosses EBs illustrated in FIGS. 11 to 13, the area of the non-contact portion 25 per unit area in the second portion p2 may differ according to the positions in the longitudinal direction D1. For example, in the state in which the absorbent article 1 is packaged by the packaging sheet 10, the area per unit area of the non-contact portion 25 in the second portion p2 can be made larger on the side away from the one end 11 of the first region R1 of the packaging sheet 10 and smaller on the side near the one end 11. In the case where the non-contact portion 25 is formed by the formation of a plurality of embosses EB illustrated in FIGS. 11 to 13, the planar view area of the embosses EB per unit area on the side away from the one end 11 can be made smaller than the planar view area per unit area of the embosses EB on the side near the one end 11.

As described above, the non-contact portion 25 disposed at least in the second portion p2 may be configured by the recessed portions DB themselves by forming the recessed portions DB (FIGS. 1 to 10) or a relatively deep portion formed between the projecting portions EB by forming the projecting portions EB (FIGS. 11 to 13).

Hereinafter, specific embodiments of the invention will be described.

### (Appendix 1)

The embodiment described in Appendix 1 is a packaging sheet for packaging an absorbent article, a first region including one end of the packaging sheet in the first direction is caused to overlap on an outer surface of a second region including another end in the first direction to enable packaging of the absorbent article, and a sealing tape is adhered over a first portion of an outer surface of the first region and a second portion of the outer surface of the second region; and, a non-contact portion that does not contact the sealing tape is formed on the outer surface of the second portion.

According to the embodiment described in Appendix 1 above, since the non-contact portion which is not in contact with the sealing tape is formed on the outer surface of the second portion to which the sealing tape is applied, the contact area between the sealing tape and the second portion of the packaging sheet, and more specifically, the contact area between the adhesive on the packaging sheet side of the sealing tape and the second portion can be reduced. This reduces the bonding force between the free portion of the sealing tape and the packaging sheet, and when the packaged absorbent article is opened with the free portion of the sealing tape, the sealing tape can be peeled off from the second region with a smaller force.

Further, according to the present embodiment, the free portion of the sealing tape can be easily peeled from the second region when the packaged absorbent article is opened without reducing the adhesion of the sealing tape. Therefore, the bonding force between the securing portion of the sealing tape and the first portion of the packaging sheet can be maintained at a high level.

### (Appendix 2)

In the embodiment described in Appendix 2, the non-contact portion is formed by forming a plurality of discontinuous recessed portions.

In the embodiment according to Appendix 2 above, the contact area between the sealing tape and the packaging sheet can be reduced over the second portion of the packaging sheet (or the free portion of the sealing tape). In addition, by forming the plurality of recessed portions in a distributed manner, the sealing tape can be smoothly peeled off even if the force is biased when a user peels the free portion.

### (Appendix 3)

In the embodiment according to Appendix 3, an area of the non-contact portion per unit area is larger on a side away from the one end than on a side close to the one end in a state in which the absorbent article is packaged.

In the embodiment according to Appendix 3 above, the contact area between the sealing tape and the packaging sheet is smaller at the beginning of peeling of the sealing tape when the packaged absorbent article is opened with the free portion of the sealing tape, and the bonding force between the sealing tape and the packaging sheet is smaller. Therefore, the force required at the beginning of peeling of the sealing tape can be reduced, and the sealing tape can be peeled off more smoothly.

### (Appendix 4)

In the embodiment according to Appendix 4, the packaging sheet is a sheet containing fibers.

In the embodiment according to Appendix 4 above, a noise generated during carrying and opening of the packaged absorbent article can be reduced and a soft touch can be provided.

### (Appendix 5)

In the embodiment according to Appendix 5, the sheet containing fibers is paper.

In the embodiment according to Appendix 5 above, the environmental load upon disposal can be reduced and the packaging sheet having a unique texture can be provided.

### (Appendix 6)

The embodiment according to Appendix 6 is a packaged absorbent article containing the packaging sheet of any one of Appendices 1 to 5 and an absorbent article.

In the embodiment according to Appendix 6 above, the packaged absorbent article that has the same effect as any of the embodiments according to Appendices 1 to 5 can be provided.

This application claims priority under Japanese Patent Application No. 2020-031875 filed February 27, 2020, and the entire contents of Japanese Patent Application No. 2020-031875 are incorporated herein by reference.

### DESCRIPTION OF THE REFERENCE NUMERALS

1 Absorbent article
10 Packaging sheet
11 One end in the longitudinal direction
12 Another end in the longitudinal direction
15 Sealing portion
20 Non-contact area forming region
25 Non-contact portion
50 Sealing tape
51 Free portion of the sealing tape
51a End of the free portion
52 Securing portion of the sealing tape
100 Packaged absorbent article
D1 First direction of the packaging sheet
D2 Second direction of the packaging sheet
DB Recessed portion
EB Projecting portion
L1 First folding line
L2 Second folding line
p1 First portion
p2 Second portion
R1 First region
R2 Second region
R3 Third region

## Claims

1. A packaging sheet for packaging an absorbent article,
wherein a first region including one end of the packaging sheet in the first direction is caused to overlap on an outer surface of a second region including another end in the first direction to enable packaging of the absorbent article, and a sealing tape is adhered over a first portion of an outer surface of the first region and a second portion of the outer surface of the second region, and
wherein a non-contact portion that does not contact the sealing tape is formed on the outer surface of the second portion.

2. The packaging sheet according to claim 1, wherein the non-contact portion is formed by forming a plurality of discontinuous recessed portions.

3. The packaging sheet according to claim 1 or 2, wherein an area of the non-contact portion per unit area is larger on a side away from the one end in which on a side close to the one end in a state that the absorbent article is packaged.

4. The packaging sheet according to any one of claims 1 to 3, wherein the packaging sheet is a sheet containing fibers.

5. The packaging sheet according to claim 4, wherein the sheet containing fibers is paper.

6. A packaged absorbent article comprising the packaging sheet of any one of claims 1 to 5 and an absorbent article.
